(19) Europäisches Patentamt
European Patent Office
Office européen des brevets

(11) Veröffentlichungsnummer: **0 080 176**
**B1**

(12) # EUROPÄISCHE PATENTSCHRIFT

(45) Veröffentlichungstag der Patentschrift: **19.02.86**

(21) Anmeldenummer: **82110678.8**

(22) Anmeldetag: **19.11.82**

(51) Int. Cl.⁴: **C 07 D 487/04,** C 07 D 491/147, A 61 K 31/505 // (C07D487/04, 249:00, 239:00),(C07D491/147, 317:00, 249:00, 239:00),(C07D491/147, 319:00, 249:00, 239:00)

(54) **Triazolochinazoline, Verfahren zu ihrer Herstellung und diese enthaltende pharmazeutische Zubereitungen.**

(30) Priorität: **25.11.81 DE 3146599**

(43) Veröffentlichungstag der Anmeldung: **01.06.83 Patentblatt 83/22**

(45) Bekanntmachung des Hinweises auf die Patenterteilung: **19.02.86 Patentblatt 86/8**

(84) Benannte Vertragsstaaten: **AT BE CH DE FR GB IT LI NL SE**

(56) Entgegenhaltungen:
**US - A - 4 053 600**
**US - A - 4 128 644**
**US - A - 4 164 578**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Schlecker, Rainer, Dr., Suedring 8, D-6719 Bissersheim (DE)**
Erfinder: **Friedrich, Ludwig, Dr., Nibelungenstrasse 8a, D-6831 Bruehl (DE)**
Erfinder: **Lenke, Dieter, Dr., Kukuleplatz 1, D-6700 Ludwigshafen (DE)**

**EP 0 080 176 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Gegenstand der vorliegenden Erfindung sind 1,2,4-Triazolo-[1,5-c]chinazolinone der allgemeinen Formel I und pharmazeutisch verträgliche Salze davon, Verfahren zu ihrer Herstellung und diese Verbindungen enthaltende pharmazeutische Zubereitungen, die als Arzneimittel bei der Behandlung von allergischen Erkrankungen verwendet werden können.

Bestimmte Triazolochinazolinone sind aus US-A- 4 053 600 als Blutdrucksenker und Entzündungshemmer bekannt.

Aus US-A- 4 164 578 und US-A 4 128 644 sind Pyrazolochinazoline bekannt, die als antiallergisches Mittel verwendet werden können. Ihre Wirkungen befriedigen jedoch nicht immer.

Es wurde nun gefunden, dass Verbindungen der Formel I

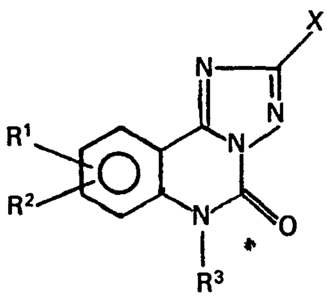

(I)

in der

X eine Carboxylgruppe ggf. in Form ihres Salzes mit einem physiologisch verträglichen Amin- oder Metallkation, oder den Rest $-\underset{\underset{O}{\|}}{C}-OR^4$, wobei $R^4$ einen Alkylrest mit 1 bis 4 C-Atomen bedeuten, und $R^1$ und $R^2$, die gleich oder verschieden sein können, ein Wasserstoffatom, ein Fluor-, Chlor- oder Bromatom oder einen Alkylrest mit 1 bis 3 C-Atomen und $R^3$ einen Alkylrest mit 1 bis 4 C-Atomen bedeuten, und ihre physiologisch verträglichen Säureadditionssalze wertvolle pharmakologische Eigenschaften, insbesondere als Antiallergika, aufweisen.

Die Herstellung der Verbindungen der allgemeinen Formel I erfolgt durch eine intramolekulare Kondensationsreaktion eines Hydrazinochinazolins der allgemeinen Formel II

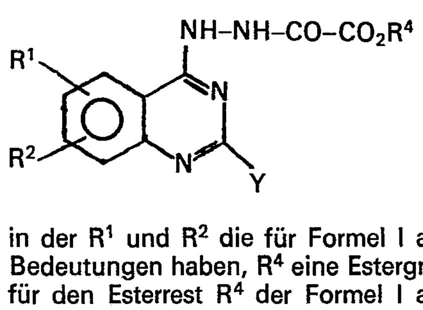

(II)

in der $R^1$ und $R^2$ die für Formel I angegebenen Bedeutungen haben, $R^4$ eine Estergruppe mit der für den Esterrest $R^4$ der Formel I angegebenen Bedeutung und Y eine Hydroxylgruppe oder ein Brom- oder Chloratom darstellt, vorzugsweise in Anwesenheit eines Dehydratisierungsmittels, insbesondere von Phosphoroxychlorid, Polyphosphorsäure oder Essigsäure, gegebenenfalls in einem inerten Lösungsmittel, wie Toluol, Chlorbenzol, Xylol oder überschüssiger Essigsäure, bei Temperaturen von 50 bis 150 °C, und zwar vorzugsweise bei der Rückflusstemperatur der Reaktionsmischung, wobei man anschliessend den erhaltenen Ester der Formel I gegebenenfalls am N-Atom in an sich üblicher Weise alkyliert, gegebenenfalls die Estergruppe verseift, die erhaltene Säure gegebenenfalls in ein Salz mit einem physiologisch verträglichen Amin- oder Metallkation überführt, oder den ursprünglich erhaltenen Ester oder die Carbonsäure weiter umsetzt, und die erhaltene Verbindung gegebenenfalls in ein pharmazeutisch verträgliches Säureadditionssalz überführt.

Zur Veranschaulichung wird ausgeführt, dass bei der erfindungsgemässen Umsetzung als primäres Cyclisierungsprodukt ein 1,2,4-Triazolo[4,3-c]chinazolinon der allgemeinen Formel Ia,

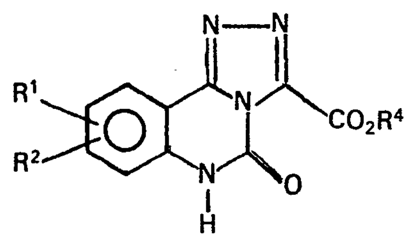

(Ia)

in der $R^1$, $R^2$ und $R^4$ die für Formel II angegebenen Bedeutungen haben, entsteht, das in einer sogenannten intramolekularen Dimroth-Umlagerung, wie beispielsweise in Aust. J. Chem. 32, 1585–93 (1979) beschrieben, zu einer Verbindung der allgemeinen Formel I isomerisiert. Die Struktur der erfindungsgemässen Verbindungen ist durch eine Röntgenstrukturanalyse gesichert. Unter den Reaktionsbedingungen geht der Substituent Y in eine Ketogruppe über.

Die Alkylierung am N-Atom in 6-Stellung erfolgt durch Umsetzung mit einem Alkylierungsmittel der Formel $R^3Z$, wobei Z für eine nucleofuge Abgangsgruppe, insbesondere ein Chlor-, Brom- oder Jodatom oder einen Sulfonsäurerest und $R^3$ für die in Formel I angegebenen Bedeutungen stehen, in Gegenwart eines säurebindenden Mittels zweckmässig bei Temperaturen von 0 °C bis 120 °C in einem inerten Lösungsmittel, wie einem niederen Alkohol mit 1 bis 4 C-Atomen oder einem Dialkylether, einem Benzolkohlenwasserstoff, wie Benzol oder Toluol, einem niederen Keton, wie Aceton, einem Dialkylformamid, wie Dimethylformamid, oder Dimethylsulfoxid. Als säurebindendes Mittel dienen insbesondere Alkali- oder Erdalkalimetallhydroxide, -carbonate, -alkoholate oder -hydride, die in stöchiometrischer Menge oder geringem Überschuss eingesetzt werden. Dabei ist Dimethylformamid als Lösungsmittel und Natriumhydrid als Base bevorzugt.

In an sich bekannter Weise können die erhaltenen Ester der Formel I einem üblichen Umesterungsverfahren entsprechend den Bedeutungen für den Rest $R^4$ unterworfen werden.

Eine weitere Umsetzung zur Herstellung von Verbindungen der allgemeinen Formel I, wobei X und $R^3$ die oben genannten Bedeutungen haben und $R^1$ und/oder $R^2$ ein Chloratom darstellen, besteht in einer elektrophilen aromatischen Substitution einer erhaltenen Verbindung der allgemeinen Formel I, in der $R^1$ und $R^2$ ein Wasserstoffatom bedeuten, nach an sich bekannten Methoden, wie sie beispielsweise in Houben-Weyl Bd. X/1, S. 471 ff, Bd. IX, S. 572 ff und Bd. V/3, S. 873, beschrieben sind. So kann die Chlorierung mit Sulfurylchlorid bei 20 °C bis 100 °C durchgeführt werden. D.h., dass bestimmte Reste $R^1$ und/oder $R_2$ nachträglich eingeführt werden können.

Die Herstellung von Verbindungen der allgemeinen Formel I, in denen X eine Carboxylgruppe darstellt, erfolgt durch Hydrolyse der entsprechenden Ester, vorzugsweise unter alkalischen Bedingungen, beispielsweise in Gegenwart eines Alkalimetallhydroxids oder Natriumhydrogencarbonats, in einem Lösungsmittel, wie Wasser, einem niederen Alkohol, Tetrahydrofuran oder Mischungen derselben. Die so erhaltenen organischen Säuren werden gegebenenfalls in ein physiologisch verträgliches Amin- oder Metallsalz überführt. Darunter versteht man insbesondere Salze der Alkalimetalle, wie Natrium und Kalium, der Erdalkalimetalle, wie Calcium, sonstiger Metalle, wie Aluminium, sowie Salze von organischen Basen, wie Morpholin, Piperidin, Mono-, Di- und Trietahnolamin oder Tris-(hydroxymethyl)aminomethan, die dem Fachmann allgemein bekannt sind.

Gegebenenfalls werden die erhaltenen erfindungsgemässen Verbindungen in das Säureadditionssalz einer physiologisch verträglichen Säure überführt. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band 10, Seiten 224 bis 225, Birkhäuser Verlag, Basel und Stuttgart, 1966, entnommen werden.

Carbonsäuren der allgemeinen Formel I können weiterhin hergestellt werden durch Hydrogenolyse der entsprechenden Benzylester nach bekannten Methoden, wie sie beispielsweise in Houben-Weyl Bd. IV/1 c S. 381 ff beschrieben sind. Die Reaktion erfolgt in Anwesenheit eines Katalysators, wie Platin, Palladium oder Nickel, zweckmässig auf einem Träger, insbesondere Kohle, in einem Lösungsmittel, wie einem niederen Alkohol, insbesondere Methanol, Essigsäure oder einem Dialkylformamid, insbesondere Dimethylformamid, zwischen Temperaturen von 0 °C bis zum Siedepunkt des Lösungsmittels, und vorzugsweise unter nur leicht erhöhtem Druck.

Die Herstellung der Ausgangsverbindungen der allgemeinen Formel II erfolgt in an sich bekannter Weise durch Kondensation von einem Hydrazinochinazolin der allgemeinen Formel III,

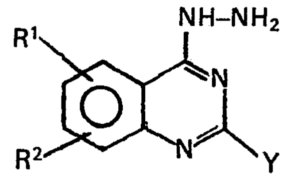

(III)

in der $R^1$, $R^2$ die für Formel I und Y die oben genannten Bedeutungen haben, mit einem Oxalsäureesterhalogenid, insbesondere Ethyloxalylchlorid, oder einem Oxalsäurediester, vorzugsweise Diethyloxalat, wobei der der Oxalestergruppe zugrundeliegende Alkohol $R^4OH$ dem Rest $R^4$ der Formel II entspricht. Bei Verwendung eines Oxalsäureesterhalogenides, bevorzugt eines Chlorids, erfolgt die Reaktion zweckmässig bei Temperaturen von −30° bis 70 °C, vorzugsweise bei Raumtemperatur, in einem inerten Lösungsmittel wie Dimethylformamid, Dioxan, Tetrahydrofuran oder Methylenchlorid. Die Umsetzung wird bevorzugt in Anwesenheit von tertiären organischen Basen, wie Triethylamin oder Pyridin, durchgeführt.

Die Umsetzung von Formel III mit Oxalsäurediestern kann mit oder ohne Lösungsmittel wie Toluol, Chlorbenzol oder Diphenylether, bei einer Temperatur von etwa 20 °C bis zur Rückflusstemperatur der Mischung durchgeführt werden. Die Oxamate der Formel I können nach bekannten Verfahren, wie sie beispielsweise in Houben-Weyl, Bd. 8, S. 526 bis 528 beschrieben sind, mit Alkoholen zu Estern mit einem anderen Rest $R^4$ umgeestert werden.

Ein weiteres Verfahren zur Herstellung von Ausgangsverbindungen der Formel II besteht in der Reaktion von einem Oxalylhydrazin der allgemeinen Formel IV,

$$H_2N{-}NH{-}CO{-}CO_2R^4 \qquad IV$$

in der $R^4$ die oben genannte Bedeutung hat, mit einem Chinazolin der allgemeinen Formel VI,

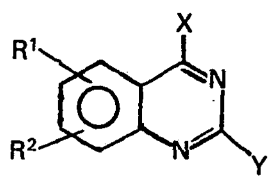

(V)

in der $R^1$, $R^2$ und Y die oben genannte Bedeutung haben und X eine nucleofuge Abgangsgruppe, bevorzugt ein Halogenatom, wie beispielsweise Chlor, sein kann. Die Reaktion wird zwischen 0° und 50 °C in einem inerten Lösungsmittel wie Ethanol, Methylenchlorid, Toluol, Tetrahydrofuran oder Dimethylformamid, vorzugsweise mit einem Überschuss an IV durchgeführt.

Die Verbindungen der allgemeinen Formel III werden zweckmässigerweise durch Kondensation von einer Verbindung der allgemeinen Formel V mit Hydrazin hergestellt. Das Verfahren wird in an sich bekannter Weise im allgemeinen

bei Temperaturen von −20 °C bis 50 °C in einem inerten Lösungsmittel, wie Dioxan, Tetrahydrofuran, Methylenchlorid oder Dimethylformamid durchgeführt.

Die Verbindungen der allgemeinen Formel IV sind bekannt und lassen sich, wie beispielsweise in Ber. dtsch. chem. Ges. 44, 776 ff (1911) beschrieben, aus Dialkyloxalaten und Hydrazin herstellen.

Die Verbindungen der allgemeinen Formel V, in der X und Y Chlor oder Brom bedeuten, lassen sich nach bekannten, beispielsweise in J. Chem. Soc. 1948, 1759 beschriebenen Methoden durch Umsetzung der entsprechenden Chinazolin-2,4-dione mit Phosphoroxychlorid oder -bromid bei Rückflusstemperatur erhalten. Die Synthesen der gleichfalls bekannten Chinazolin-2,4-dione aus Alkaliisocyanat und Anthranilsäure oder Anthranilsäureestern bzw. aus Harnstoff und Anthranilsäurederivaten sind in einer Übersicht in W.L.F. Amarego, Quinazolines, S. 116 bis 218, Wiley 1967, New York zusammengestellt.

Ein weiteres Verfahren zur Herstellung von Verbindungen der allgemeinen Formel I besteht in der Umsetzung von 3-(o-Aminophenyl)triazolen der Formel VI

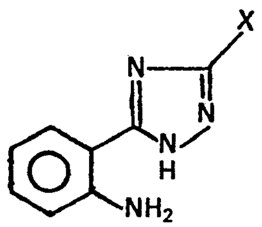

(VI)

mit Kohlensäurederivaten, wie Phosgen, Chlorameisensäureestern oder N,N'-Carbonyldiimidazol. Die Reaktion wird vorzugsweise in Anwesenheit eines inerten organischen Lösungsmittels, wie Methylenchlorid, Benzol, Ether oder Tetrahydrofuran, durchgeführt bei Temperaturen zwischen 0 °C und der Siedetemperatur des Lösungsmittels.

Weiterhin kann diese Cyclisierung durch Umsetzen mit Harnstoff bei 150 bis 200 °C oder mit Alkaliisocyanaten in Essigsäure durchgeführt werden.

Die Verbindungen der Formel VI lassen sich nach bekannten Methoden durch Reduktion der entsprechenden Nitroderivate herstellen. Die Synthese der 3-(o-Nitrophenyl)triazole erfolgt nach den üblichen, beispielsweise in Heterocyclic Compounds Vol. 7, S. 425 ff., Wiley 1961 angegebenen Verfahren der Triazolchemie.

Die erfindungsgemässen Verbindungen und ihre physiologisch verträglichen Salze sind Pharmaka, die als wertvolle Heilmittel bei der Behandlung von allergischen Erkrankungen des Respirations-, des Gastro-Intestinaltraktes und der Haut, beispielsweise bei allergischem Asthma, allergischer Rhinitis oder Nahrungsmittel-Allergien, verwendet werden können.

Die antiallergische Wirkung wurde an Ratten getestet. Zur Prüfung wurde das Modell der passiv cutanen Anaphylaxie (PCA) verwendet. Narkotisierte männliche Ratten (100 bis 140 g) werden durch intradermale Injektionen (rasierte Rükkenhaut) von 0,1 ml eines Ovalbumin-Antiserums sensibilisiert. Nach einer Sensibilisierungsperiode von ca. 48 Stunden erfolgt die Behandlung (intraperitoneale oder orale Applikation) mit unterschiedlichen Dosierungen (10 Tiere/Dosis) der Prüfsubstanzen. 15 oder 20 Minuten nach der Behandlung wird eine Antigen/Evansblau-Mischung (10 mg/kg Ovalbumin in 2%iger Evansblau-Lösung) den Versuchstieren intravenös injiziert. 30 Minuten später werden die Tiere getötet, die Rückenhaut abgezogen und auf der inneren Oberfläche der Durchmesser der kreisförmigen Blaufärbung gemessen. Die Grösse der Farbflecke unbehandelter Kontrolltiere ist standardisierbar. Antiallergisch wirksame Substanzen verkleinern dosisabhängig den Durchmesser der Farbflecke. Als ED 50% wird die Dosis angegeben, die im Vergleich zu medikamentös unbehandelten Kontrolltieren den Durchmesser der Farbflecke um 50% herabsetzt.

Ausser der antiallergischen Wirkung wurde die akute Toxizität an Gruppen von je 2 NMRI-Mäusen, Gewicht 20 bis 26 g, bei intraperitonealer Applikation und an je 2 Sprague-Dawley-Ratten, Gewicht 120 bis 150 g bei oraler Applikation ermittelt. Die Bestimmung der LD 50 erfolgte an Gruppen von je 10 NMRI-Mäusen, Gewicht 20 bis 26 g, nach intraperitonealer Applikation. Die Nachbeobachtungszeit betrug 14 Tage.

Die erfindungsgemässen Verbindungen sind antiallergisch hochwirksam. Aus der Tabelle 1 geht hervor, dass die neuen Verbindungen nach oraler Gabe 4,4- (Bsp. 17) bis 1,2- (Bsp. 27) mal wirksamer sind als die Vergleichsverbindung Pirquinozol (J. Pharmacol. exp. Therap. 213, 432 bis 436, 1980; JAMA, 242, 1912, 1979). Die Referenzsubstanz Cromolyne ist in dieser Versuchsanordnung bis 100 mg/kg p.o. unwirksam.

Hinsichtlich der akuten Toxizität unterscheiden sich die neuen Verbindungen wenig oder nur unwesentlich von den Vergleichsverbindungen. Aufgrund der besseren Wirksamkeit ergibt sich jedoch ein grösserer Abstand zwischen den toxischen und den wirksamen Dosen.

Tabelle 1
Antiallergische Wirkung nach oraler Gabe. PCA. Ratte

| Bsp. | Hemmung der PCA Ratte ED 50% mg/kg | R.W- |
|---|---|---|
| 13 | 0,518 | 2,29 |
| 14 | 0,707 | 1,68 |
| 17 | 0,271 | 4,39 |
| 25 | 0,617 | 1,92 |
| 20 | 0,723 | 1,64 |
| 28 | 0,876 | 1,36 |
| 27 | 1,00 | 1,19 |
| Pirquinozol | 1,19 | 1,00 |
| Cromolyne | > 100 | – |

R.W.: relative Wirksamkeit, Pirquinozol = 1,00

Auf Grund der Tabelle 1 sind besonders bevorzugt die Bedeutungen Carboethoxy für X, Wasserstoff, Chlor und Methyl für $R^1$ und $R^2$ und Methyl und Ethyl für $R^3$.

Für die therapeutische Anwendung werden Einzeldosen von 0,1 bis 100 mg verwendet.

Gegenstand der vorliegenden Erfindung sind demnach auch therapeutische Mittel oder Zubereitungen, die neben pharmazeutisch üblichen Träger- und Verdünnungsmitteln und Hilfsmitteln eine Verbindung der Formel I als Wirkstoff enthalten, sowie Verbindungen zur Verwendung bei der Behandlung von allergischen Erkrankungen.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Weiterhin kommen Inhalate und parenterale Zubereitungen, wie Injektionslösungen, in Betracht.

Die galenischen Applikationsformen, fest oder flüssig, werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wässrigen oder nichtwässrigen Trägern, Netzmitteln, Dispergiermitteln, Emulgatoren und/oder Konservierungsmitteln, verarbeitet werden (vgl. R. Voigt, Lehrbuch der pharmazeutischen Technologie, VEB Verlag Volk und Gesundheit, Berlin 1977).

Herstellung von Ausgangsverbindungen

Beispiel A

2-Chlor-4-hydrazinochinazolin

Eine Suspension von 86,5 g (0,43 Mol) 2,4-Dichlorchinazolin in 1500 ml Methylenchlorid wird bei Raumtemperatur unter Eiskühlung und starkem Rühren tropfenweise mit 87 g (1,74 Mol) Hydrazinhydrat versetzt. Nach 8 Std. bei Raumtemperatur wird der Niederschlag abgesaugt, mit Wasser gewaschen und getrocknet. Ausb. 78,4 g (92%) 2-Chlor-4-hydrazinochinazolin, Fp. > 280 °C.

Nach dem gleichen Verfahren werden die folgenden Verbindungen hergestellt (die Schmelzpunkte liegen mit über 280 °C an der Messgrenze des Schmelzpunktapparates):

4-Hydrazino-2,6,8-trichlorchinazolin, Fp, > 280 °C, Ausb. 66%

2-Chlor-4-hydrazino-6-methylchinazolin, Fp. > 280 °C, Ausb. 73%

2,6-Dichlor-4-hydrazinochinazolin, Fp. > 280 °C, Ausb. 80%

2-Chlor-5-fluoro-4-hydrazinochinazolin, Fp. > 280 °C, Ausb. 79%

Beispiel B

2-Chlor-4-N(N'-ethyloxalylhydrazino)chinazolin

16,9 g (0,087 Mol) 2-Chlor-4-hydrazinochinazolin werden in 400 ml abs. Methylenchlorid/14 ml Triethylamin suspendiert und unter Eiskühlung mit 14,2 g (0,104 Mol) Oxalsäuremonoethylesterchlorid versetzt. Die Mischung wird über Nacht bei Raumtemperatur gerührt, der Niederschlag abgesaugt, mit Wasser gewaschen und getrocknet.

Ausb. 14,6 g (57%), Fp. 211 °C

$C_{12}H_{11}N_4O_3Cl$ (295)

| | | | | |
|---|---|---|---|---|
| Ber. | 48,9 C | 3,8 H | 19,0 N | 12,0 Cl |
| Gef. | 48,9 C | 4,0 H | 19,3 N | 12,2 Cl |

Analog werden aus den entsprechenden Ausgangsverbindungen und Oxalestern hergestellt:

4-N(N'-Ethyloxalylhydrazino)-2,6-8-trichlorochinazolin, Fp. 154 bis 158 °C, Ausbeute 52%

2,6-Dichlor-4-N(N'-ethyloxalylhydrazino)-chinazolin, Ausbeute 56%

2-Chlor-4-N(N'-ethyloxalylhydrazino)-5-fluorochinazolin, Ausbeute 56%

2-Chlor-4-N(N'-ethyloxalylhydrazino)-6-methylchinazolin, Ausbeute 59%

2-Chlor-4-N(N'-isoamyloxalylhydrazino)-chinazolin, Fp. 209 bis 211 °C, Aub. 38%

2-Chlor-4-N(N'-cyclohexyloxalylhydrazino)-chinazolin, Fp. 220 bis 221 °C, Ausb. 40%

4-N(N'-Benzyloxalylhydrazino)-2-chlorchinazolin, Fp. 217 bis 218 °C, Ausb. 66%

Herstellung von erfindungsgemässen Verbindungen:

Beispiel 1

1,2,4-Triazolo[1,5-c]chinazolin-5-on-2-carbonsäureethylester

57 g (0,19 Mol) 2-Chlor-4-N(N'-ethyloxalylhydrazino)chinazolin werden in 900 ml Essigsäure 4 Std. unter Rückfluss gekocht. Das Lösungsmittel wird abdestilliert, der Rückstand mit Wasser gerührt und getrocknet. 36 g (73%), Fp. 279 °C

| | | | | |
|---|---|---|---|---|
| $C_{12}H_{10}N_4O_3$ (258) | Ber. 55,8 C | 3,9 H | 21,7 N | |
| | Gef. 55,9 C | 4,3 H | 21,8 N | |

In gleicher Weise werden jeweils aus der Chlorverbindung hergestellt:

Beispiel 2

9-Methyl-1,2,4-triazolo[1,5-c]chinazolin-5-on-2-carbonsäureethylester

Fp. 286 bis 287 °C, Ausb. 72%

$C_{13}H_{12}N_4O_3$ (272)

| | | | |
|---|---|---|---|
| Ber. | 57,4 C | 4,4 H | 20,6 N |
| Gef. | 57,0 C | 4,4 H | 20,3 N |

Beispiel 3

7,9-Dichlor-1,2,4-triazolo[1,5-c]chinazolin-5-on-2-carbonsäureethylester

Fp. 265 bis 269 °C, Ausb. 48%

$C_{12}H_8Cl_2N_4O_3$ (327)

| | | | | |
|---|---|---|---|---|
| Ber. | 44,0 C | 2,4 H | 17,1 N | 21,7 Cl |
| Gef. | 43,8 C | 2,6 H | 17,5 N | 21,2 Cl |

Beispiel 4

9-Chlor-1,2,4-triazolo[1,5-c]chinazolin-5-on-2-carbonsäureethylester

Fp. 280 °C, Ausb. 60%
$C_{12}H_9ClN_4O_3$ (292)
Ber. 49.3 C 3,1 H 19,1 N 12,1 Cl
Gef. 49,3 C 3,2 H 19,5 N 12,2 Cl

Beispiel 5
10-Fluoro-1,2,4-triazolo[1,5-c]chinazolin-5-on-2-carbonsäureethylester,
Fp. 290 bis 291 °C, Ausb. 31%
$C_{12}H_9FN_4O_3$ (276)
Ber. 52,2 C 3,3 H 20,3 N 6,9 F
Gef. 52,1 C 3,3 H 20,0 N 6,8 F

Beispiel 6
6-Methyl-1,2,4-triazolo[1,5-c]chinazolin-5-on-2-carbonsäureethylester

Zu einer Suspension von 0,053 Mol NaH in 100 ml abs. Dimethylformamid werden bei Raumtemperatur 12,0 g (0,047 Mol) 1,2,4-Triazolo[1,5-c]chinazolin-5-on-2-carbonsäureethylester in 100 ml abs. Dimethylformamid getropft. Nach 2 Std. wird die Mischung mit 7,5 g (0,053 Mol) Methyljodid versetzt, über Nacht bei Raumtemperatur gerührt und in 400 ml Eiswasser gegossen. Der Niederschlag wird abgesaugt und getrocknet.

6,6 g (52%), Fp. 258 °C.
$C_{13}H_{12}N_4O_3$ (272) Ber. 57,3 C 4,4 H 20,6 N
Gef. 57,5 C 4,8 H 20,6 N

Nach dem obigen Verfahren werden unter anderen die folgenden Verbindungen hergestellt, wobei mit den entsprechenden Alkyljodiden, Benzylbromid, Allylbromid oder Methoxyethoxymethylchlorid umgesetzt wird.

Beispiel 7
6-Ethyl-1,2,4-triazolo[1,5-c]chinazolin-5-on-2-carbonsäureethylester
Fp. 207 bis 209 °C, Ausb. 38%
$C_{14}H_{14}N_4O_3$ (286) Ber. 58,7 C 4,9 H 19,6 N
Gef. 58,5 C 4,9 H 19,8 N

Beispiel 8
6-Propyl-1,2,4-triazolo[1,5-c]chinazolin-5-on-2-carbonsäureethylester,
Fp- 203 bis 204 °C, Ausb. 42%
$C_{15}H_{16}N_4O_3$ (300) Ber. 60,0 C 5,4 H 18,7 N
Gef. 59,6 C 5,3 H 18,8 N

Beispiel 9
6-Ethyl-9-methyl-1,2,4-triazolo[1,5-c]chinazolin-5-on-2-carbonsäureethylester,
Fp. 175 bis 178 °C, Ausb. 43%
$C_{15}H_{16}N_4O_3$ (300) Ber. 60,0 C 5,4 H 18,7 N
Gef. 59,6 C 5,4 H 18,4 N

Beispiel 10
9-Chlor-6-methyl-1,2,4-triazolo[1,5-c]chinazolin-5-on-2-carbonsäureethylester
Fp. 250 bis 254 °C, Ausb. 92%
$C_{13}H_{11}ClN_4O_3$ (306)
Ber. 50,9 C 3,6 H 18,3 N 11,6 Cl
Gef. 51,0 C 4,1 H 17,9 N 11,3 Cl

Beispiel 11
10-Fluor-6-methyl-1,2,4-triazolo[1,5-c]chinazolin-5-on-2-carbonsäureethylester
Fp. 290 °C, Ausb. 61%
$C_{13}H_{11}FN_4O_3$ (290)
Ber. 53,8 C 3,8 H 19,3 N 6,6 F
Gef. 53,4 C 3,8 H 19,1 N 6,4 F

Beispiel 12
6,9-Dimethyl-1,2,4-triazolo[1,5-c]chinazolin-5-on-2-carbonsäureethylester
Fp. 220 nis 222 °C, Ausb. 49%
$C_{14}H_{14}N_4O_3$ (286) Ber. 58,7 C 4,9 H 19,6 N
Gef. 59,0 C 5,2 H 19,2 N

Beispiel 13
6,8,9-Trimethyl-1,2,4-triazolo[1,5-c]chinazolin-5-on-2-carbonsäureethylester
Fp. 276 bis 278 °C, Ausb. 94%
$C_{15}H_{16}N_4O_3$ (300) Ber. 60,0 C 5,4 H 18,7 N
Gef. 59,7 C 5,5 H 18,5 N

Beispiel 14
8,9-Dimethyl-6-ethyl-1,2,4-triazolo[1,5-c]chinazolin-5-on-2-carbonsäureethylester
Fp. 230 bis 233 °C, Ausb. 58%
$C_{16}H_{18}N_4O_3$ (314) Ber. 61,1 C 5,8 H 17,8 N
Gef. 61,0 C 5,7 H 17,8 N

Beispiel 15
8,9-Dimethyl-6-propyl-1,2,4-triazolo[1,5.]chinazolin-5-on-2-carbonsäureethylester
Fp. 218 bis 221 °C, Ausb. 86%
$C_{17}H_{20}N_4O_3$ (328) Ber. 62,2 C 6,1 H 17,1 N
Gef. 62,5 C 6,4 H 16,9 N

Beispiel 16
6-Propyl-1,2,4-triazolo[1,5-c]chinazolin-5-on-2-carbonsäure

3,5 g (0,012 Mol) 6-Propyl-1,2,4-triazolo [1,5-c] chinazolin- 5-on- 3-carbonsäureethylester wurden in 50 ml Wasser mit 1,1 g $NaHCO_3$ versetzt und 8 Std. unter Rückfluss erhitzt. Die Reaktionsmischung wurde filtriert, mit verd. HCl angesäuert und der Niederschlag abgesaugt und getrocknet.

1,0 g (32%), Fp. 233 bis 235 °C,
$C_{13}H_{12}N_4O_3$ (272) Ber. 57,4 C 4,4 H 20,6 N
Gef. 57,0 C 4,4 H 20,7 N

Beispiel 17
1,2,4-Triazolo[1,5-c]chinazolin-5-on-2-carbonsäure

5 g (15,6 mmol) 1,2,4-Triazolo[1,5-c]chinazolin-5-on-2-carbonsäurebenzylester wurden in 250 ml Methanol gelöst und nach Zugabe von 1 g 5% Pd/C in einer Hydrierapparatur unter Normaldruck bis zur Beendingung der Wasserstoffaufnahme gerührt. Der Niederschlag wurde abgesaugt, in 50 ml heissem Dimethylformamid gelöst, die Lösung filtriert und das Lösungsmittel abdestilliert. Der Rückstand wurde mit Methanol digeriert und getrocknet.

Ausb. 1,3 g (36%), Fp. 290 °C

$C_{10}H_6N_4O_3$ (230) Ber. 52,2 C 2,6 H 24,3 N
Gef. 52,0 C 2,9 H 24,2 N

Beispiel 18

6-Methyl-1,2,4-triazolo[1,5-c]chinazolin-5-on-2-carbonsäure durch Hydrogenolyse gemäss Beispiel 17

Fp. 290 °C, Ausb. 34%

$C_{11}H_8N_4O_3 \cdot H_2O$ (262)

Ber. 50,4 C 3,8 H 21,4 N

Gef. 50,6 C 4,0 H 21,3 N

Beispiele für pharmazeutische Zubereitungen

Tabletten

| | |
|---|---|
| a) Wirkstoff | 0,100 g |
| Stearinsäure | 0,010 g |
| Traubenzucker | 1,890 g |
| | 2,000 g |

| | |
|---|---|
| b) Wirkstoff | 0,020 g |
| Stearinsäure | 0,020 g |
| Traubenzucker | 1,960 g |
| | 2,000 g |

Die Bestandteile werden in üblicher Weise zu Tabletten der vorstehend angegebenen Zusammensetzung verarbeitet.

Inhalationsaerosol

| | |
|---|---|
| Wirkstoff | 1,00 Teile |
| Sojalecithin | 0,20 Teile |
| Treibgasmischung (Frigen 11, 12 und 114) ad | 100,00 Teile |

Die Zubereitung wird vorzugsweise in Aerosolbehälter mit Dosierventil abgefüllt. Der einzelne Hub wird so bemessen, dass eine Dosis von 5 bis 20 mg Wirkstoff abgegeben wird.

Ampullen (Injektionslösungen)

| | |
|---|---|
| Wirkstoff | 50,0 Gew.-Teile |
| Natriumpyrosulfit | 1,0 Gew.-Teile |
| Dinatriumsalz der Ethylendiamintetraessigsäure | 0,5 Gew.-Teile |
| Natriumchlorid | 8,5 Gew.-Teile |
| doppelt destilliertes Wasser ad | 1000,0 Gew.-Teile |

Der Wirkstoff und die Hilfsmittel werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 50 mg Wirkstoff.

**Patentansprüche**

1. Verbindungen der Formel I

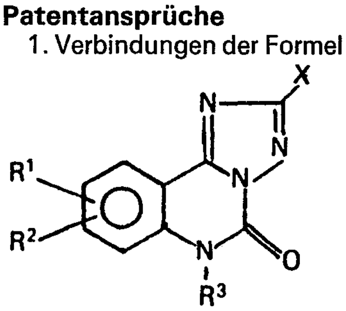

(I)

in der X eine Carboxylgruppe ggf. in Form ihres Salzes mit einem physiologisch verträglichen Amin- oder Metallkation, oder den Rest $-\underset{\underset{O}{\|}}{C}-OR^4$, wobei $R^4$ einen Alkylrest mit 1 bis 4 C-Atomen bedeutet, und $R^1$ und $R^2$, die gleich oder verschieden sein können, ein Wasserstoffatom, ein Fluor-, Chlor- oder Bromatom oder einen Alkylrest mit 1 bis 3 C-Atomen und $R^3$ einen Alkylrest mit 1 bis 4 C-Atomen bedeuten.

2. Verbindungen der Formel I nach Anspruch 1, in denen X einen Carboethoxyrest, $R^1$ und $R^2$ ein Wasserstoffatom, ein Chloratom oder Methyl und $R^3$ Methyl oder Ethyl bedeuten.

3. 6-Ethyl- 9-methyl- 1,2,4- triazolo [1,5-c]-chinazolin- 5-on -2-carbonsäureethylester und dessen physiologisch verträglichen Säureadditionssalze.

4. 6-Methyl-1,2,4-triazolo [1,5-c] chinazolin-5-on-2-carbonsäureethylester und dessen physiologisch verträglichen Säureadditionssalze.

5. Verfahren zur Herstellung der Verbindungen der Formel I nach Anspruch 1, dadurch gekennzeichnet, dass man eine intramolekulare Kondensationsreaktion eines Hydrazinochinazolins der allgemeinen Formel II

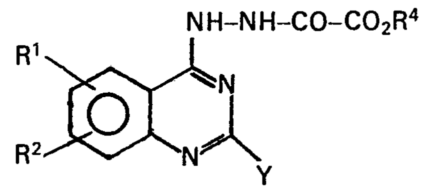

(II)

in der $R^1$ und $R^2$ die für Formel I angegebenen Bedeutungen haben, $R^4$ mit der für den Esterrest $R^4$ der Formel I angegebenen Bedeutung und Y eine Hydroxylgruppe oder ein Brom- oder Chloratom darstellt, vorzugsweise in Anwesenheit eines Dehydratisierungsmittels, gegebenenfalls in einem inerten Lösungsmittel bei Temperaturen von 50 bis 150 °C durchführt und man den erhaltenen Ester der Formel I gegebenenfalls am N-Atom in an sich üblicher Weise alkyliert, gegebenenfalls die Estergruppe verseift, die erhaltene Säure gegebenenfalls in ein Salz mit einem physiologisch verträglichen Amin- oder Metallkation überführt, und die erhaltene Verbindung gegebenenfalls in ein pharmazeutisch verträgliches Säureadditionssalz überführt.

6. Verfahren zur Herstellung von Verbindungen der Formel I nach Anspruch 5, in denen X einen Carboethoxyrest, $R^1$ und $R^2$ ein Wasserstoffatom, ein Chloratom oder Methyl und $R^3$ Methyl oder Ethyl bedeuten.

7. Therapeutisches Mittel, enthaltend eine Verbindung der Formel I nach Anspruch 1 als Wirkstoff neben üblichen pharmazeutischen Träger- und Verdünnungsmitteln und pharmazeutisch-technischen Hilfsmitteln.

8. Verbindung der Formel I nach Anspruch 1 zur Verwendung bei der Behandlung von allergischen Erkrankungen.

**Revendications**

1. Composés de la formule I

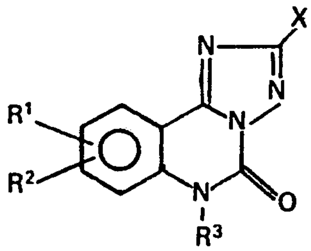

(I),

dans laquelle

X représente un groupe carboxyle, éventuellement sous forme d'un sel avec un cation amine ou métal physiologiquement compatible, ou un groupe de la formule $-\underset{\underset{O}{\|}}{C}-OR^4$, où $R^4$ désigne un radical alkyle en $C_1$ à $C_4$, $R^1$ et $R^2$, qui peuvent être identiques ou différents, désignent chacun un atome d'hydrogène, de fluor, de chlore ou de brome ou un radical alkyle en $C_1$ à $C_3$ et $R^3$ désigne un radical alkyle en $C_1$ à $C_4$.

2. Composés de la formule I selon la revendication 1, pour lesquels X représente un groupe carbéthoxy, $R^1$ et $R^2$ désignent chacun un atome d'hydrogène ou de chlore ou un radical méthyle et $R^3$ désigne un radical méthyle ou éthyle.

3. Le 6-éthyl-9-méthyl-1,2,4-triazolo [1,5-c]-quinazoline-5-one-2-carboxylate d'éthyle et ses sels d'addition d'acides physiologiquement compatibles.

4. Le 6-méthyl-1,2,4-triazolo [1,5-c] quinazoline-5-one-2-carboxylate d'éthyle et ses sels d'addition d'acides physiologiquement compatibles.

5. Procédé de préparation de composés de la formule I selon la revendication 1, caractérisé en ce que l'on soumet une hydrazino-quinazoline de la formule générale

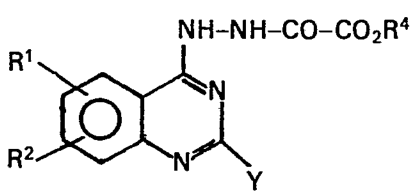

(II)

dans laquelle $R^1$ et $R^2$ possèdent les significations définies pour la formule I, $R^4$ possède la signification de $R^4$ du groupe ester de la formule I et Y désigne un groupe hydroxyle ou un atome de brome ou de chlore, à des températures de 50 à 150 °C, éventuellement dans un solvant inerte et de préférence en présence d'un agent deshydratant, à une réaction de condensation intramoléculaire, l'ester obtenu de la formule I pouvant être alkylé de manière connue sur l'atome d'azote, le groupe ester éventuellement saponifié, l'acide obtenu pouvant le cas échéant être transformé en un sel avec un cation amine ou métal physiologiquement acceptable et le composé obtenu en un sel d'addition d'un acide physiologiquement compatbile.

6. Procédé de préparation de composés de la formule I selon la revendication 5, pour lesquels X représente un groupe carbéthoxy, $R^1$ et $R^2$ désignent chacun un atome d'hydrogène ou de chlore ou un radical méthyle et $R^3$ désigne un radical méthyle ou éthyle.

7. Composition pharmaceutique, contenant un composé de la formule I selon la revendication 1 en tant que principe actif à côté de diluants et de matières supports pharmaceutiques habituels et d'adjuvants pharmaceutico-techniques usuels.

8. Composé de la formule I selon la revendication 1, utilisé pour le traitement d'affections allergiques.

**Claims**

1. A compound of the formula I

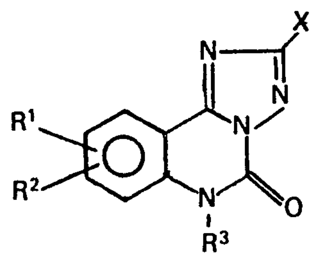

(I),

where X is carboxyl which may be in the form of a salt with a physiologically tolerated amine or metal cation, or is $-\underset{\underset{O}{\|}}{C}-OR^4$, where $R^4$ is alkyl of 1 to 4 carbon atoms, and $R^1$ and $R^2$ may be identical or different and are each hydrogen, fluorine, chlorine or bromine, or alkyl of 1 to 3 carbon atoms, and $R^3$ is alkyl of 1 to 4 carbon atoms.

2. A compound of the formula I as claimed in claim 1, where X is carboethoxy, $R^1$ and $R^2$ are each hydrogen, chlorine or methyl, and $R^3$ is methyl or ethyl.

3. Ethyl 6-ethyl-9-methyl-1,2,4-triazolo [1,5-c]- quinazolin-5-one-2-carboxylate and its physiologically tolerated addition salts with acids.

4. Ethyl 6-methyl-1,2,4-triazolo [1,5-c] quinazolin-5-one-2-carboxylate and its physiologically tolerated addition salts with acids.

5. A process for the preparation of a compound of the formula I as claimed in claim 1, wherein a hydrazinoquinazoline of the general formula II

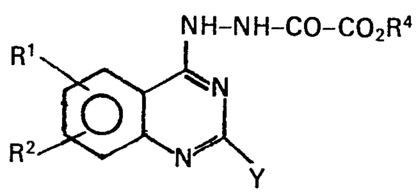

(II)

where $R^1$ and $R^2$ have the meanings given for formula I, $R^4$ has the meaning given for the ester radical $R^4$ of the formula I, and Y is hydroxyl, bromine or chlorine, is subjected to an intramolecular

condensation reaction, preferably in the presence of a dehydrating agent, in the presence or absence of an inert solvent, at from 50 to 150 °C, and the resulting ester of the formula I is, if required, alkylated in a conventional manner at the N atom, the ester group is, if required, hydrolysed, and the acid obtained is, if necessary, converted into a salt with a physiologically tolerated amine or metal cation, and the compound obtained is converted, if required, into a pharmaceutically tolerated addition salt with an acid.

6. A process for the preparation of a compound of the formula I as claimed in claim 5, where X is carboethoxy, $R^1$ and $R^2$ are each hydrogen, chlorine or methyl, and $R^3$ is methyl or ethyl.

7. A therapeutic agent containing a compound of the formula I as claimed in claim 1, as active ingredient, as well as conventional pharmaceutical carriers and diluents and pharmaceutical auxiliaries.

8. A compound of the formula I as claimed in claim 1 for use in the treatment of allergic disorders.